# EUROPÄISCHE PATENTSCHRIFT

(11) **EP 3 897 376 B1**
(45) Veröffentlichungstag und Bekanntmachung des Hinweises auf die Patenterteilung: **28.01.2026**
(21) Anmeldenummer: 19835400.3
(22) Anmeldetag: 20.12.2019
(51) Int. Cl.: A61B 5/296, A61B 5/256

(54) **TEXTILELEKTRODE**
TEXTILE ELECTRODE
ÉLECTRODE TEXTILE

(30) Priorität: 21.12.2018 DE 102018133336; 21.12.2018 DE 102018133335
(43) Veröffentlichungstag der Anmeldung: 27.10.2021
(73) Patentinhaber: Otto Bock Healthcare Products GmbH, 1110 Wien (AT)
(72) Erfinder: WAGNER, Sonja, 1180 Wien (AT); SCHERB, Alice, Stockerau (AT); LUNZER, Walter, 1150 Wien (AT); AMSÜSS, Sebastian, 1180 Wien (AT); KOPPE, Mario, 37115 Duderstadt (DE)
(74) Vertreter: Gramm, Lins & Partner Patent- und Rechtsanwälte PartGmbB
(86) Internationale Anmeldenummer: PCT/EP2019/086619
(87) Internationale Veröffentlichungsnummer: WO 2020/127934

(56) Entgegenhaltungen:
- EP-A1- 2 671 506
- WO-A1-2008/022482
- DE-U1- 202006 007 226
- US-A1- 2012 071 015
- US-A1- 2017 196 514
- PITOU SAMUEL ET AL: "Embroidered Electrodes for Control of Affordable Myoelectric Prostheses", 2018 IEEE INTERNATIONAL CONFERENCE ON ROBOTICS AND AUTOMATION (ICRA), IEEE, 21 May 2018 (2018-05-21), pages 1812 - 1817, XP033403490, DOI: 10.1109/ICRA.2018.8461066
- GUANGLIN LI ET AL: "Performance of electromyography recorded using textile electrodes in classifying arm movements", ENGINEERING IN MEDICINE AND BIOLOGY SOCIETY,EMBC, 2011 ANNUAL INTERNATIONAL CONFERENCE OF THE IEEE, IEEE, 30 August 2011 (2011-08-30), pages 4243 - 4246, XP032319623, ISBN: 978-1-4244-4121-1, DOI: 10.1109/IEMBS.2011.6091053
- TAO D ET AL: "Real-time performance of textile electrodes in electromyogram pattern-recognition based prosthesis control", BIOMEDICAL AND HEALTH INFORMATICS (BHI), 2012 IEEE-EMBS INTERNATIONAL CONFERENCE ON, IEEE, 5 January 2012 (2012-01-05), pages 487 - 490, XP032449110, ISBN: 978-1-4577-2176-2, DOI: 10.1109/BHI.2012.6211624

## Beschreibung

Die Erfindung betrifft eine Textilelektrode mit einem flächigen Grundkörper aus einem elektrisch nicht leitenden Material mit einer im angelegten Zustand einer Hauptoberfläche zugewandten Innenseite und einer der Innenseite gegenüberliegenden Außenseite.

Textilelektroden werden zur Auflage auf die Haut eines Patienten verwendet, um im angelegten Zustand elektrische, insbesondere myoelektrische Signale aufzunehmen und an eine Auswerteeinrichtung, gegebenenfalls unter Vorschaltung eines Verstärkers, weiterzuleiten. Alternativ kann über eine Textilelektrode ein elektrisches Signal oder ein elektrischer Impuls über die Hautfläche auf den Patienten übertragen werden, um eine Stimulierung der Muskulatur zu bewirken. Ebenfalls ist es möglich, über die Elektrode zu überwachende Kenngrößen eines Patienten zu erfassen. Der Grundkörper aus einem elektrisch nicht leitenden Material ist so beschaffen, dass ein elektrisches Signal oder ein elektrischer Impuls, das durch den Grundkörper hindurch über einen Leiter zu einer Kontaktfläche geleitet werden, nicht oder nur vernachlässigbar verfälscht wird. Der Grundkörper ist bevorzugt flexibel ausgebildet, um eine flächige Anlage auf der Hautoberfläche zu ermöglichen.

Zur Steuerung von Prothesen oder Orthesen werden myoelektrische Signale von der Hautoberfläche abgenommen, um diese als Steuerungssignale zur Steuerung von Antrieben oder Verstelleinrichtungen von Orthesen oder Prothesen zu nutzen. Dazu werden in der Regel Domelektroden oder Saugschaftelektroden verwenden, die auf der Hautoberfläche positioniert werden. Bei Saugschaftelektroden wird eine metallische, elektrisch leitende Kontaktfläche mit einem Verstärker in einem Kunststoffgehäuse in einer Ausnehmung in einem Liner oder in einem Prothesenschaft positioniert. Aufgrund der Aufbauhöhe des Gehäuses ist es notwendig, den Liner und/oder den Außenschaft mit einer entsprechenden Aussparung für das Gehäuse zu versehen. Die Positionierung der Saugschaftelektrode ist insbesondere bei einer Anordnung in einer Ausnehmung in einem formstabilen Außenschaft nicht veränderbar. Durch die Notwendigkeit eines Ausschnittes wird die Verwendung einer Vakuumschafttechnologie erschwert.

Domelektroden bestehen aus halbkugelartigen Kontaktflächen aus Metall, beispielsweise Titan. Von den Kontaktflächen führen Kabel zu Verstärkern und zu einer Steuerungseinrichtung, in der das myoelektrische Signal ausgewertet und verarbeitet wird. Gegebenenfalls wird das Signal digitalisiert und anschließend zur Steuerung eines Aktuators verwendet. Domelektroden sind ebenfalls in einer Ausnehmung in einem Liner zu integrieren und sind einerseits ortsständig und andererseits aufgrund der punktuellen Ausgestaltung der Kontaktfläche nur schwer zu positionieren.

Die WO 2016/131936 A1 betrifft eine Vorrichtung oder System und ein Verfahren zur Ermittlung von Reizen an einem Nutzer. Die Reize können elektrisch Stimulationsreize und auch haptische Reize wie Vibrationen umfassen. Insbesondere sollen Stimulationsimpulse zur Stimulation biologischer Gewebe wie Muskeln oder Nerven auf den Nutzer übertragen werden. Hierzu werden Stimulationselektroden auf ein Textil gestickt oder gemoost oder vorab auf ein Trägermaterial aufgebracht, das wiederum mit dem Textil verbunden, insbesondere genäht, gestickt, geklebt, gelasert oder verschweißt wird.

Die DE 10 2014 108 316 A1 betrifft eine Fahrradhose zur muskulären Stimulation der Oberschenkelmuskulatur mit wenigstens einem Paar integrierter, textilbasierter Kontaktelektroden und einem Körpersensor, der für die Aufnahme einer biologischen Rückantwort zur Entwicklung der aktiven Bewegungsphase ausgestattet ist.

Die US 2017/0196514 A1 betrifft einen Sensor zum Messen physiologischer elektrischer Signale, mit einer erster Textilelektrode, die einen detektierenden Textilabschnitt zum Detektieren physiologischer elektrischer Signale und einen peripheren Textilabschnitt direkt angrenzend an den Detektierabschnitt umfasst, wobei der detektierende Textilabschnitt einen elektrisch leitfähigen Detektieroberflächenbereich aufweist, der dazu bestimmt ist, mit der Haut eines Individuums in Kontakt zu kommen. Der Sensor weist eine erste elektrische Verbindung auf, die konfiguriert ist, um die erste Textilelektrode elektrisch mit einem ersten elektrischen Verbinder zu verbinden, wobei die erste Textilelektrode eine dreidimensionale Textilstruktur aufweist, die durch Verweben von Kettfäden und Schussfäden hergestellt ist. Der Erfassungsabschnitt umfasst eine obere Textilschicht, deren obere Oberfläche sich über die Erfassungsfläche erstreckt, und eine darunter angeordnete untere Textilschicht. Die obere Schicht und untere Schicht sind entlang einer Umfangsverbindungslinie damit verbunden, um einen durch die Verbindungslinie definierten Hohlraum zu erzeugen und einen Bereich außerhalb der Verbindungslinie zu definieren, der den peripheren Textilabschnitt umfasst. Der Hohlraum ist durch ein Füllmaterial ausgefüllt, so dass der detektierende Textilabschnitt gegenüber dem peripheren Textilabschnitt in der Höhe hervorsteht.

Die DE 20 2006 007 226 U1 beschreibt Textilelektrode mit einem ersten textilen Flächengebilde zur Auflage auf die Haut eines Patienten und einem zweiten textilen Flächengebilde, das vom ersten textilen Flächengebilde beabstandet angeordnet ist. Das erste textile Flächengebilde umfasst mindestens einen Elektrodenbereich, der aus einem elektrisch leitfähigen Faden gebildet ist. Zwischen dem ersten textilen Flächengebilde und dem zweiten textilen Flächengebilde verläuft mindestens ein Abstandsfaden. Das zweite textile Flächengebilde ist derart elastisch verformbar ausgebildet, dass sich eine einer elastischen Verformung des zweiten textilen Flächengebildes entgegenwirkende Rückstellkraft über den Abstandsfaden auf den Elektrodenbereich überträgt.

WO 2008/022482 A1 betrifft eine Textilelektrodenvorrichtung, insbesondere für mehrkanalige Elektrostimulation oder elektrophysiologischen Messungen mit einer Textillage, die an der körperzugewandten Seite mit einer für Hautkontakt ausgelegten Elektrodengruppe versehen ist, wobei die Elektrodengruppe mehrere auf die Textillage gestickte Stickelektroden umfasst, die jeweils mit einer elektrischen Zuleitung verbunden sind. Die Zuleitung ist als gestickte Leiterbahn auf einer zweiten Textillage ausgebildet, die auf der körperabgewandten Seite der ersten Textillage angeordnet ist, wobei eine jede Stickelektrode mit der zugeordneten Leiterbahn in einem Kontaktierungsbereich flächig kontaktiert ist.

Die EP 2 671 506 A1 beschreibt eine dreidimensionale Textilelektrode zur Einbettung in eine Gestrick-Grundschicht zur Messung personenbezogener elektrischer Potenziale eines Anwenders. Die Textilelektrode weist ein rohrförmiges Trägerelement mit einer Längsachse auf, ein rohrförmiger, leitfähiger Bereich mit einer Längsachse besteht aus einem leitfähigem Garn und Elastan und umgibt das Trägerelement radial. Zwei nicht leitfähige Bereiche grenzen den leitfähigen Bereich in einer axialen Längsrichtung ab, wobei eine Silikon-Isolierschicht an den Seiten oder auf der Hinterseite des rohrförmigen leitfähigen Bereiches aufgebracht.

Die Veröffentlichung "Embroidered Electrodes For Control Of Affordable Myoelectric Electrodes" 2018 IEEE International Conference On Robotics And Automation, 21. Mai 2018, Seiten 1812 - 1817, XP033403490, Pitou et al.) beschreibt eine aufgestickte Textilelektrode aus Metallfäden mit einem Druckknopf. Dabei werden die metallischen Fäden mit einer Nähmaschine aufgestickt.

Die Veröffentlichung "Performance of Electromyography Recorded Using Textile Electrodes In Classifying Arm Movements", Engineering In Medicine And Biology Society, EMBC, 2011 Annual International Conference Of The IEEE, 30. August 2011, Seiten 4243 - 4246, XP032319623, Li et al.) beschreibt eine Textilelektrode unter Verwendung von Kupfer-basierten, Nickel beschichteten leitenden Materialien. Die Textilelektroden sind auf einem Band angeordnet, das aus Gummi und Nylonmaterial hergestellt worden ist.

Die Veröffentlichung "Real-Time Performance Of Textile Electrodes In Electromyogram Pattern-Recognition Based Prosthesis Control", Biomedical And Health Informatic, 2012 IEEE-EMBS International Conference, 5. Januar 2012, Seiten 487 - 490, XP032449110, Tao et al.) beschreibt Textilelektroden, die im Siebdruck mit leitfähiger Tinte auf Textil erhalten wurden. Die Textilelektroden sind auf zwei Bändern befestigt, die um eine Gliedmaße herum gelegt werden.

Problematisch ist weiterhin die Erfassung myoelektrischer Signale, die durch eine Aktivierung der Muskulatur entstehen und durch Elektroden aufgenommen werden sollen.

Kleidungsstücke und orthopädietechnische Einrichtungen, die an einem Patienten getragen werden, weisen zunehmend elektronische Komponenten auf, die darin integriert oder dauerhaft daran befestigt sind. Bei solchen sogenannten "Smart Textiles" mit den elektronischen Einrichtungen wie Verstärkern oder Steuerungseinrichtungen oder Auswerteeinrichtungen zur Auswertung von Sensorsignalen besteht die Problematik, dass diese Produkte nicht waschbar sind oder aber dass bei einem Ausfall der elektronischen Einrichtungen das gesamte Kleidungsstück oder die gesamte orthopädietechnische Einrichtung ausgetauscht werden muss. Insbesondere Tenside in Waschmitteln greifen auch bei wasserdichten Ausführungen die elektronischen Komponenten an und können diese insbesondere zusammen mit den mechanischen Belastungen bei einem Waschvorgang zerstören.

Herkömmliche Steckverbindungen, Knöpfe oder Druckknöpfe zur lösbaren Verbindung der elektronischen Einrichtung mit dem Textil sind meist relativ groß und voluminös und daher unkomfortabel und die Kontakte befinden sich separiert in jeweils einem Druckknopf, sodass ein Zusammenführen aller Kontakte in nur einem Druckknopf nicht möglich ist. Druckknöpfe bestehen meist aus Metall, so dass jeder Druckknopf eine Kontaktfläche bildet. Darüber hinaus ist eine eindeutige Zuordnung von Kontakten nicht immer gegeben.

Die Erfindung betrifft daher auch eine Kontaktierungsvorrichtung zur lösbaren Befestigung elektrischer oder elektronischer Komponenten an einem flexiblen Grundkörper sowie eine orthopädietechnische Einrichtung mit einem flexiblen Grundkörper aus einem Textil oder Schaumstoff mit einer solchen Kontaktierungsvorrichtung.

Aufgabe der vorliegenden Erfindung ist es daher, eine Elektrode bereitzustellen, die einen gegenüber herkömmlichen Elektroden vereinfachten Aufbau aufweist, in der Lage ist, myoelektrische Signale aufzunehmen und einfach und vielseitig einsetzbar ist.

Erfindungsgemäß wird diese Aufgabe durch eine Textilelektrode mit den Merkmalen des Hauptanspruches gelöst. Vorteilhafte Ausgestaltung und Weiterbildungen der Erfindung sind in den Unteransprüchen, den Figuren sowie der Beschreibung offenbart.

Die Textilelektrode mit einem flächigen Grundkörper aus einem elektrisch nicht leitenden Material mit einer im angelegten Zustand einer Hautoberfläche zugewandten Innenseite und einer der Innenseite gegenüberliegenden Außenseite sieht vor, dass zumindest eine myoelektrische Signale aufnehmende Elektrode auf der Innenseite angeordnet oder ausgebildet ist, wobei die Elektrode zumindest einen elektrisch leitenden Faden aufweist oder daraus ausgebildet ist, der eine Kontaktfläche ausbildet. Die Kontaktfläche ist auf zumindest einer Bondingfolie ausgebildet, die über den Außenumfang der Kontaktfläche hinaussteht. Die Erfindung sieht vor, dass die Kontaktfläche auf einem Textilzuschnitt ausgebildet ist, auf dem zumindest eine Bondingfolie angeordnet ist. Diese Bondingfolie steht über den Außenumfang des Textilzuschnittes hinaus und wird mit dem Grundkörper verbunden. Der Textilzuschnitt entspricht dann bei vorteilhafterweise im Wesentlichen der Kontur eines Ausschnittes in dem Grundkörper und füllt diesen Ausschnitt vorteilhafterweise vollständig oder nahezu vollständig aus. Die Bondingfolie oder die Bondingfolien stehen über den Rand des Ausschnittes in Richtung auf den Grundkörper über und legen die Elektrode an dem gewünschten Punkt des Grundkörpers fest. Die Außenseite der Elektrode ist durch eine weitere Bondingfolie abgedeckt.

Die Kontaktfläche der Elektrode zur Aufnahme myoelektrischer Signale kann zur Ausbildung flacher und weniger auftragender Elektroden aus zumindest einem elektrisch leitenden Faden bestehen oder einen solchen aufweisen, wobei diese Elektrode beispielsweise auch in einer textilen Innenbeschichtung eines Liners angeordnet werden kann. Das Elektrodenareal eines Mustererkennungssystems kann mit einer Vielzahl solcher myoelektrischer Elektroden auf textiler Basis in Linern verwendet werden, so dass ein Orthopädietechniker bei der Anpassung der Elektrode im Zusammenhang mit der Versorgung eines Patienten mit Prothesen oder Orthesen nicht jede einzelne Elektrode in eine Prothesenschaft oder in eine Aufnahme für ein Körperglied einbauen und verschrauben muss.

Die Elektrode kann auf dem Grundkörper aufgestickt, aufgenäht, eingewebt, eingestrickt, gedruckt, gebonded, aufgeschweißt, eingeschweißt oder aufgeklebt werden oder alternativ als integraler Bestandteil des Grundkörpers ausgebildet sein. Ein integraler Bestandteil des Grundkörpers ist eine Elektrode oder ein Elektrodenbereich dann, wenn der elektrisch leitende Faden als ein strukturwesentliches Element des Grundkörpers ausgebildet ist, beispielsweise bei einem gewebten Grundkörper als Kettfaden oder Schussfaden ausgebildet ist. Wird die Elektrode aufgedruckt, kann diese in einem additiven Herstellverfahren wie 3D-Druck oder im Siebdruckverfahren geschehen. Durch die Verbindung mit dem flexiblen Grundkörper ist es möglich, dass die aus elektrisch leitenden Fäden bestehenden Elektroden oder die elektrisch leitende Fäden aufweisende Elektroden sich bei Muskelkontraktionen zusammen mit dem Grundkörper verformen und an der Krümmung des Körpers und sich verändernden Formen angepasst bleiben, statt sich wie bei starren Elektroden von der Hautoberfläche partiell abzuheben. Durch die Ausbildung mit oder aus einem Faden ist es möglich, einen saugfähigen Grundkörper als Träger zu verwenden, so dass auch bei starken Feuchtigkeitsansammlungen, beispielsweise aufgrund von Schweißbildung, Kurzschlüsse zwischen Kontaktflächen oder Kontaktpunkten verhindert oder vermindert werden könnten.

Eine Weiterbildung der Erfindung sieht vor, dass die Elektrode mit einem metallischen Ober- und/oder Unterfaden als elektrisch leitenden Faden ausgebildet ist, ggf. kann der Unterfaden aus einem metallischen Material und der Oberfaden aus einem nichtmetallischen Material ausgebildet sein, wodurch das Vernähen des metallischen Fadens ggf. erleichtert wird. Der metallische, elektrisch leitende Faden kann als rein metallischer Faden, beispielsweise aus Edelstahl, Silber, Gold, Kupfer, Platin oder Titan ausgebildet sein. Ebenfalls ist es möglich, den metallischen Faden mit einem Kunststoffkern und einer metallischen Beschichtung oder einem anderen leitenden Material wie z.B. Graphit zu versehen oder den Faden aus diesem Material auszubilden. Silber als Material ist ebenfalls grundsätzlich geeignet, sowohl zur Ausbildung eines reinmetallischen Fadens als auch zur Beschichtung, hat jedoch den Nachteil einer Oxidationsneigung. Goldfäden oder Goldbeschichtungen haben den Nachteil vergleichsweise hoher Kosten.

Eine Variante der Erfindung sieht vor, dass die Elektrode mit einem Lege- und/oder Nadelfaden als elektrisch leitenden Faden ausgebildet ist, um eine Kontaktfläche herzustellen. Alternativ oder ergänzend kann die Elektrode als als Fläche verstrickter elektrisch leitender Faden oder mit einem Kett- und/oder Schussfaden als elektrisch leitenden Faden ausgebildet sein.

Der nichtmetallische Oberfaden kann aus einem Kunststoff- oder Naturmaterial ausgebildet sein, gegebenenfalls kann der nichtmetallische Oberfaden aus einem hochfesten, insbesondere elastischen Material ausgebildet sein, um eine ausreichende Gegenspannung beim Vernähen und eine ausreichende Festigkeit bereitzustellen.

Eine Weiterbildung der Erfindung sieht vor, dass mehrere Kontakte nebeneinander und elektrisch voneinander getrennt an dem Grundkörper angeordnet sind, wodurch es möglich ist, eine Elektrodenanordnung mit einer Masseelektrode oder Kennungselektrode vorzugsweise in der Mitte zwischen zwei Signalelektroden auszubilden.

Von der jeweiligen Kontaktfläche der Elektrode kann ein elektrischer Leiter zu einer elektrischen oder elektronischen Komponente, einer Steckerbasis oder einem Stecker an oder in dem Grundkörper geführt sein. Es ist beispielsweise möglich, die Kontaktfläche der Elektrode so auszubilden, dass bei der Ausgestaltung der Elektrode nicht durch alle Lagen oder durch die komplette Dicke des Grundmaterials hindurchgestochen oder gedruckt wird, so dass auf der Außenseite kein elektrisch leitender Faden angeordnet ist. Grundsätzlich ist es jedoch auch möglich, dass durch den Grundkörper mit dem elektrisch leitenden Faden durchgestochen wird. Die Leitung kann als einfacher Vorstich innerhalb des Gewebes oder als geführte Leitung in einem Zwischenraum zwischen der Innenseite und Außenseite des Grundkörpers ausgebildet sein. An dem der Kontaktfläche abgewandten Ende des elektrischen Leiters kann eine Anschlussstelle oder ein Kontaktpunkt angeordnet oder ausgebildet sein, um die Elektrode mit einem lösbaren Kontakt zu verbinden, beispielsweise um einen Verstärker und/oder eine Steuerungseinrichtung mit den myoelektrischen Signalen der Elektrode zu versorgen.

Eine Weiterbildung der Erfindung sieht vor, dass die Kontaktfläche aus einem Zuschnitt ausgebildet ist, die an dem Grundkörper befestigt ist. Der Zuschnitt ist bevorzugt ebenfalls ein Textil und kann aus einem größeren Textilmaterial ausgeschnitten, ausgestanzt oder auf eine andere Art und Weise herausgetrennt werden. Ebenfalls ist es möglich, dass der Zuschnitt einen Kern oder eine Basis aus einem Nichttextil aufweist, das bestickt, umwickelt, umwebt oder benäht ist, so dass aus dem elektrisch leitenden Faden eine entsprechende Kontaktfläche ausgebildet werden kann. Dieser separate Zuschnitt mit zumindest einem elektrisch leitenden Faden oder mit einer aus einem elektrisch leitenden Faden hergestellten Kontaktfläche wird anschließend auf dem Grundkörper befestigt, insbesondere aufgenäht, aufgeklebt oder aufgeschweißt. Bei einer Vernähung des Zuschnittes wird bevorzugt ein elektrisch leitender Faden verwendet, der gleichzeitig als Leiter zu einem Stecker, einer Steckerbasis oder einem Kontaktpunkt zu einer elektrischen oder elektronischen Komponente dient.

Eine Weiterbildung der Erfindung sieht vor, dass dem Zuschnitt ein Volumenelement hinterlegt ist, so dass sich eine Aufwölbung in Richtung auf die Hautoberfläche ergibt. Das Volumenelement ist vorzugsweise flexibel ausgebildet, insbesondere elastisch, um eine gleichmäßige und flächenmäßig möglichst große Anpressung und Auflage auf der Hautoberfläche zu ermöglichen, ohne den Tragekomfort zu verringern. Das Volumenelement kann vollständig umnäht, verklebt, verschweißt, oder dergleichen sein, insbesondere mit elektrisch leitenden Faden umnäht sein. Es besteht ebenfalls die Möglichkeit, dass ein zweilagiger Zuschnitt mit einem zwischen den beiden Lagen angeordneten Volumenelement auf dem Grundkörper aufgebracht wird, beispielsweise aufgeklebt, aufgenäht, aufgestickt oder dergleichen. Die beiden Lagen des Zuschnittes können auch miteinander verklebt sein, beispielsweise über eine Bondingfolie.

Der Grundkörper kann aus einem Abstandstextil mit einem Obertextil und einem Untertextil und dazwischen angeordneten Stützfäden ausgebildet sein, wobei die Stützfäden das Obertextil und des Untertextil aneinander festlegen und zueinander beabstandet halten. Alternativ kann der Grundkörper aus einem mehrlagigen Textilsystem bestehen oder aus einem Gewebe oder Gestrick, das bevorzugt ausreichend dick ist, um einen Leiter, der von einer Kontaktfläche wegführt, aufzunehmen.

Bevorzugt ist die Kontaktfläche auf einer Seite von zwei Lagen Bondingfolie aufgenäht, wobei die Klebeseiten der Bondingfolien einander zugewandt sind. Die Kontaktfläche ist auf der den Klebeseiten abgewandten Seiten der Bondingfolie ausgebildet und kann mit einem Oberfaden aus einem Edelstahlgarn oder einem anderen elektrisch leitenden Faden und einem Unterfaden, ebenfalls aus einem elektrisch leitenden Material, beispielsweise Edelstahl hergestellt ist, aufgenäht sein. Alternativ oder ergänzend kann der elektrisch leitenden Faden oder können die elektrisch leitenden Fäden mit einem anderen, oben beschriebenen Verfahren verarbeitet werden, um die Kontaktfläche auszubilden. Zum Verbinden der Elektrode wird der Grundkörper mit einer Durchgangsöffnung versehen, die bevorzugt so groß wie der Außenumfang um die Kontaktflächen ist. Die überstehende Bondingfolie wird mit den Klebeschichten auf der jeweiligen Innenseite oder Außenseite des Grundkörpers aufgeklebt und somit eine dauerhafte Verbindung mit dem Grundkörper hergestellt. Ein elektrischer Leiter von der Kontaktfläche zu einer weiteren elektrischen oder elektronischen Komponente kann innerhalb des Grundkörpers geführt sein.

Von der Kontaktfläche kann ein elektrischer Leiter zu einem auf der Außenseite des Grundkörpers oder eines Textilzuschnittes angeordneten, elektrisch leitenden Anschlusspunkt führen, von dem ausgehend eine Steckerkontaktierung stattfinden kann, um die myoelektrischen Signale weiterzuleiten, zu verstärken und/oder einer Steuerung zuzuführen.

Aufgabe der vorliegenden Erfindung ist es zudem, eine Kontaktierungsvorrichtung sowie eine orthopädietechnische Einrichtung mit einer Kontaktierungsvorrichtung herzustellen, die eine sichere Verbindung und eine leichte Trennbarkeit der elektronischen Komponente von einem Grundkörper ermöglichen.

Erfindungsgemäß wird diese Aufgabe durch eine Kontaktierungsvorrichtung zur lösbaren Befestigung elektrischer oder elektronischer Komponenten an einem flexiblen Grundkörper, mit einer Basis, die an dem Grundkörper festlegbar ist und eine Öffnung aufweist, die von dem Grundkörper weg zeigt, mit zumindest einer elektrisch leitenden Kontaktfläche, die an der Basis in der Öffnung angeordnet ist und mit einem elektrischen Leiter verbunden ist, der an oder in dem Grundkörper positionierbar ist, mit einem Steckerelement, an dem zumindest eine Steckerkontaktfläche angeordnet oder ausgebildet ist, die korrespondierend zu der Kontaktfläche an der Basis ausgebildet ist und mit einem elektrischen Leiter verbunden ist, die Öffnung der Basis dient zur Aufnahme des Steckerelementes und das Steckerelement und die Basis weisen korrespondierende Formschlusselemente auf, die in einem gefügten Zustand von Steckerelement und Basis eine formschlüssige Verriegelung entgegen einer Verlagerung aus der Öffnung heraus bewirken, wobei das Steckerelement und die Basis unter einer Vorspannung miteinander verrasten sowie einer orthopädietechnischen Einrichtung mit einem flexiblen Grundkörper aus einem Textil oder Schaumstoff gelöst.

Die Kontaktierungsvorrichtung zur lösbaren Befestigung elektrischer oder elektronischer Komponenten an einem flexiblen Grundkörper sieht eine Basis vor, die an dem Grundkörper festlegbar ist und eine Öffnung aufweist, die von dem Grundkörper weg zeigt, wenn die Basis an dem Grundkörper festgelegt ist. Die Kontaktierungsvorrichtung weist weiterhin zumindest eine elektrisch leitende Kontaktfläche auf, die an der Basis in der Öffnung angeordnet ist und mit einem elektrischen Leiter verbunden ist, der an oder in dem Grundkörper positionierbar ist. Ein Steckerelement, an dem zumindest eine Steckerkontaktfläche angeordnet oder ausgebildet ist, die korrespondierend zu der Kontaktfläche an der Basis ausgebildet ist und mit einem elektrischen Leiter verbunden ist, ist der Kontaktierungsvorrichtung zugeordnet. Die Öffnung der Basis dient zur Aufnahme des Steckerelementes, wobei das Steckerelement und die Basis korrespondierende Formschlusselemente aufweisen, die in einem gefügten Zustand von Steckerelement und Basis eine formschlüssige Verriegelung entgegen einer Verlagerung aus der Öffnung heraus bewirken. Dabei verrasten das Steckerelement und die Basis unter einer Vorspannung miteinander, sodass die Kontaktierungsvorrichtung in einer Richtung arretiert, wobei die Arretierungsrichtung und Vorspannung bevorzugt senkrecht zu der Öffnungsrichtung verläuft, also bevorzugt in einer Ebene, die parallel zu der Ebene des Grundkörpers verläuft, wenn dieser flach ausgebildet ist.

Die elektrischen Leiter, die mit der Kontaktfläche oder den Kontaktflächen an oder in der Basis verbunden sind, können in dem montierten Zustand der Kontaktierungsvorrichtung an oder insbesondere innerhalb des Grundkörpers verlegt sein, beispielsweise in Freiräumen, Kanälen oder Zwischenräumen in dem Grundkörper, beispielsweise zwischen zwei Textilschichten oder zwischen einem Obertextil und einem Untertextil eines Abstandsgewirkes.

Da die Kontaktierungsvorrichtung an dem Körper eines Nutzers tragbar ausgebildet sein kann, ist die Öffnung der Basis in Richtung auf den Grundkörper bevorzugt abgedichtet oder geschlossen, zumindest im Wesentlichen geschlossen ausgebildet, um ein Eindringen von Feuchtigkeit, beispielsweise Schweiß, von der Körperseite aus zu vermindern oder zu vermeiden. Dadurch wird verhindert, dass die Kontaktflächen korrodieren. Die Leiter von den Kontaktflächen können durch den Grundkörper hindurch zu weiteren elektrischen oder elektronischen Komponenten geleitet werden, beispielsweise zu Sensoren oder Elektroden zur Aufnahmen myoelektrischer Signale. Die Abdichtung muss nicht notwendigerweise wasserdicht und/oder luftdicht ausgeführt sein, eine weitgehende Verhinderung des Eindringens von Feuchtigkeit ist ausreichend.

An der Basis kann ein Auflagerand zur Auflage auf den Grundkörper angeordnet oder ausgebildet sein. Da die Basis mit der Öffnung eine Tiefe aufweist, ist es bevorzugt vorgesehen, dass die Kontaktierungsvorrichtung in einer Ausnehmung innerhalb des Grundkörpers eingebettet wird, beispielsweise in einen Ausschnitt des Grundkörpers oder eine Aushöhlung. Um eine oberflächenbündige Positionierung der Basis an dem Grundkörper zu erleichtern oder zu ermöglichen, wird ein Auflagerand an der Basis ausgebildet, der um die Öffnung herum angeordnet ist, sodass die Positionierung ermöglicht und erleichtert wird. Der Auflagerand kann verschweißbar oder verklebbar mit dem Grundkörper ausgebildet sein und beispielsweise aus einem mit dem Grundkörper verschweißbaren Material bestehen oder auf der dem Grundkörper zugewandten Seite mit einem Klebstoff versehen oder versehbar sein. Die Verschweißung oder Verklebung kann vollumfänglich ausgebildet sein, alternativ ist nur eine teilweise Verklebung oder Verschweißung mit dem Grundkörper vorhanden.

Eine Weiterbildung der Erfindung sieht vor, dass die Öffnung parallel zueinander und/oder rechtwinklig zueinander angeordnete Seitenkanten aufweist, um eine rechteckige oder nahezu rechteckige Öffnung bereitzustellen, in der das Steckerelement mit einer korrespondierenden Formgebung aufgenommen werden kann. Durch eine solche Formgebung des Umfanges der Öffnung ist es möglich, dass das Steckerelement eindeutig orientiert innerhalb der Öffnung gehalten werden kann, sodass eine präzise Kontaktierung durch Inkontaktbringen von Kontaktflächen erleichtert wird.

An dem Steckerelement und der Basis können Ausrichteinrichtungen zur eindeutigen Orientierung von Steckerelement und Basis zueinander angeordnet oder ausgebildet sein, beispielsweise durch eine Formgebung der Öffnung, die eine falsche Kontaktierung ausschließt. Dazu sind nicht rotationsymmetrische Formgebungen oder im Wesentlichen klappsymmetrische Formgebungen mit Vorsprüngen und korrespondierenden Ausnehmungen an dem Steckerelement und der Basis vorgesehen. Ebenfalls ist es möglich, dass die Formschlusselemente zur Festlegung von Steckerelementen an der Basis so ausgebildet sind, dass nur eine eindeutige Orientierung und damit ein sicherer und eine falsche Kontaktierung vermeidende formschlüssige Festlegung der Basis an dem Steckerelement möglich ist.

Bevorzugt sind drei Kontaktflächen an der Basis und drei dazu korrespondierende Steckerkontaktflächen an dem Steckerelement angeordnet, um Elektroden innerhalb des Grundkörpers über eine entsprechende Kontaktierung durch die Leiter mit den Kontaktflächen zu verbinden. Die Elektroden weisen in der Regel zwei Signalkontaktflächen und eine Erdungskontaktfläche auf, die bevorzugt zwischen den beiden Signalkontaktflächen angeordnet ist. Zusammen mit der eindeutigen Zuordnung von Steckerelement zur Basis und der eindeutigen Kontaktierung wird eine sichere und fehlverpolungsfreie Festlegung des Steckerelementes an der Basis und damit eine Kontaktierungsmöglichkeit der Elektrode mit einer elektrischen oder elektronischen Einrichtung, wie Steuerungseinrichtung, Auswerteeinrichtung oder Verstärker möglich.

In einer Ausgestaltung der Erfindung sind die Steckerkontaktflächen an einer Stirnseite des Steckerelementes angeordnet, um zusammen mit der Einführbewegung eine Kontaktierung der Steckerkontaktflächen mit den Kontaktflächen an der Basis oder innerhalb der Basis zu ermöglichen. Die Steckerkontaktflächen sind bevorzugt an der Stirnseite eines Formschlusselementes angeordnet, das in der Basis einrastet und eine Entnahmebewegung oder ein Entfernen des Steckerelementes aus der Öffnung senkrecht zu der Ebene, in der die Öffnung liegt, verhindert. Die Formschlusselemente können vorteilhafterweise so ausgebildet sein, dass sie als vorspringende Zunge und korrespondierende Ausnehmung ausgebildet sind, wobei die Zunge oder Vorsprünge vorzugsweise an dem Steckerelement angeordnet ist, die Ausnehmung bevorzugt innerhalb der Öffnung. Durch Einschwenken und Herunterdrücken des Steckerelementes ist es möglich, die Steckerkontaktfläche mit der Kontaktfläche innerhalb der Basis in Berührung zu bringen und einen elektrischen Kontakt oder elektrische Kontakte zur Weiterleitung elektrischer Signale herzustellen. Durch ein auf der der Zunge gegenüberliegenden Seite angeordnetes oder auch quer dazu wirkendes Klipselement oder Federelement ist es möglich, eine entsprechende Verriegelung gegen eine Entfernungsbewegung oder eine Ausschwenkbewegung bereitzustellen. Durch eine Federkraft oder eine Vorspannung, die in Richtung auf die Stirnseite des Steckerelementes wirkt, wird eine zusätzliche Anpresskraft der Steckerkontaktfläche auf die Kontaktfläche innerhalb der Basis bereitgestellt.

Um die Öffnung herum kann eine Dichtung angeordnet oder ausgebildet sein, mit der es möglich ist, die Kontaktierung innerhalb der Öffnung oder Basis gegen Feuchtigkeit, Schmutz und andere Umwelteinflüsse zu schützen. Bevorzugt ist die Dichtung dergestalt angeordnet oder ausgebildet, dass sie in Zusammenwirkung mit dem Steckerelement die Öffnung abdichtet, zumindest ein Eindringen von Feuchtigkeit und/oder Schmutz vermindert.

An dem Stecker kann ein Verstärker zur Verstärkung eines elektrischen Signals aufgenommen werden, das von den Kontaktflächen der Basis zu einer anderen elektrischen oder elektronischen Einrichtung geleitet werden soll.

Eine Weiterbildung der Erfindung sieht vor, dass der Basis ein Deckel zum Verschließen der Öffnung zugeordnet ist, beispielsweise um die Öffnung während eines Waschvorganges zu verschließen, um die Kontakte vor Lösungsmitteln oder Waschmitteln zu schützen, wenn das Steckerelement und die an dem Steckerelement über von dem Steckerelement wegführenden Leiter angeordnete elektrische oder elektronische Komponente entfernt sind. Dadurch wird einerseits die Waschbarkeit des Grundköpers im montierten Zustand mit der Kontaktierungsvorrichtung gewährleistet und andererseits ein wirksamer Schutz der Kontaktstellen innerhalb der Basis erreicht.

Die orthopädietechnische Einrichtung sieht einen flexiblen Grundkörper aus einem Textil oder Schaumstoff vor, an oder in der eine Kontaktierungsvorrichtung wie sie oben beschrieben worden ist, angeordnet oder integriert ist.

In dem Grundkörper können die Leiter von der Kontaktfläche oder den Kontaktflächen der Basis eingebettet sein, sodass einerseits der Leiter vor mechanischen und anderen Umwelteinflüssen geschützt ist und andererseits der Tragekomfort der orthopädietechnischen Einrichtung nicht beeinträchtigt wird.

Sensoren oder Elektroden, die mit dem Leiter verbunden sind, können in dem Grundkörper integriert oder daran festgelegt sein, beispielsweise aufgeklebt, aufgenäht, damit verschweißt oder durch Aufsticken, Aufweben oder dergleichen ausgebildet sein.

Die Sensoren oder Elektroden können über eine Bondingfolie auf dem Grundkörper festgelegt werden, sodass ein Schutz oder eine zumindest weitgehende Abdichtung gegenüber dem Gewebe oder dem Schaumstoff des Grundkörpers bei gleichzeitiger Festlegung an dem Grundkörper erreicht werden kann.

Die Basis kann mit dem Grundkörper verklebt oder verschweißt oder auf eine andere Art und Weise daran befestigt werden. Ebenfalls kann die Basis über eine Bondingfolie mit Kontaktpunkten oder Durchgangsöffnungen für die Leiter mit dem Grundkörper verbunden sein, sodass zunächst Leiter von Sensoren oder Elektroden mit den Kontaktpunkten an einer Bondingfolie verbunden sind und die Basis anschließend über diese Bondingfolie mit den Kontaktpunkten in eine dauerhafte elektrische Verbindung gebracht wird.

Nachfolgend werden Ausführungsbeispiele der Erfindung anhand der beigefügten Figuren näher erläutert. Es zeigen:
- Figur 1: einen Grundkörper mit drei Elektroden;
- Figur 2: einen Grundkörper mit einem auf der Außenseite angeordneten Steckerelement;
- Figur 3: einen Grundkörper mit Textilelektroden auf der Innenseite;
- Figur 4: der Grundkörper gemäß Figur 3 von der Außenseite gesehen;
- Figuren 5 bis 7: Elektroden mit Zuschnitten;
- Figur 8: ein Elektrodenpad mit doppellagiger Bondingfolie;
- Figur 9: ein Pad gemäß Figur 8 vor der Montage;
- Figur 10: eine schematische Schnittdarstellung der Verbindung von Elektrode mit Grundkörper;
- Figur 11: Elektrodenanordnung mit Kontaktpunkten auf der Außenseite;
- Figur 12: eine Steckerbasis und eine Bondingfolie;
- Figur 13: die zusammengebauten Komponenten der Figuren 11 und 12;
- Figur 14 -: eine orthopädietechnische Einrichtung mit einer Kontaktierungseinrichtung;
- Figur 15: eine Detailansicht der Figur 14;
- Figur 16-: eine demontierte Kontaktierungseinrichtung in Draufsicht;
- Figur 17-: eine Sensoranordnung;
- Figur 18 -: eine teilmontierte Basis;
- Figur 19 -: eine Untenansicht einer orthopädietechnischen Einrichtung mit Elektroden und Sensoren;
- Figur 20 -: eine Teilansicht einer orthopädietechnischen Einrichtung mit einer gefügten Kontaktierungseinrichtung;
- Figur 21 -: eine Detailansicht einer Basis mit Verbindungselementen;
- Figur 22 -: eine Draufsicht auf ein Steckelement;
- Figur 23 -: eine Untenansicht eines Steckerelementes;
- Figur 24 -: eine Seitenansicht eines Steckerelementes;
- Figur 25 -: eine schematische Schnittdarstellung eines Steckerelementes;
- Figur 26 -: ein Steckerelement vor der Kontaktierung;
- Figur 27 -: eine Draufsicht und eine Untenansicht einer Basis;
- Figur 28 -: eine schematische Schnittdarstellung eines Fügevorganges; sowie
- Figur 29 -: eine schematische Darstellung der Komponenten und deren Zusammenbau.

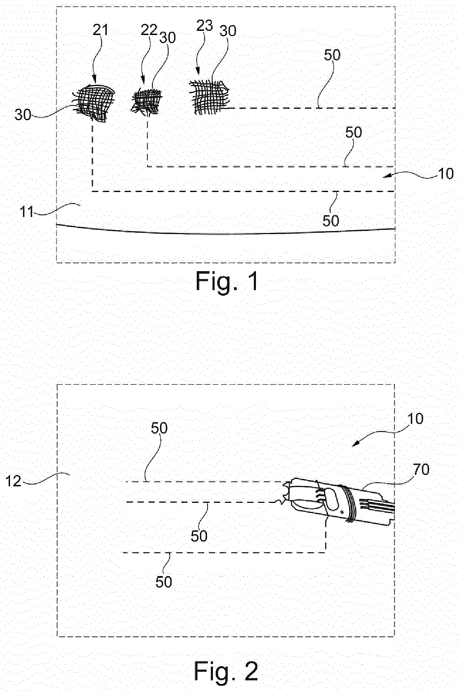

Figur 1 zeigt einen Grundkörper 10 in einer Teilansicht mit einer sichtbaren Innenseite 11, auf deren Oberfläche insgesamt drei Elektroden 21, 22, 23 ausgebildet sind. Die Elektroden 21, 22, 23 sind aus jeweils einem elektrisch leitenden Faden 30 ausgebildet, der im dargestellten Ausführungsbeispiel aus Edelstahlgarn besteht und auf der Oberseite der Innenfläche 11 eingewebt ist, sodass der elektrisch leitende Faden 30 ein Kontaktfeld oder eine Kontaktfläche 40 ausbildet, das oder die zur Auflage auf der Haut eines Patienten geeignet ist. Es können auch mehrere elektrisch leitenden Fäden 30 oder ein Gemisch aus Fäden verschiedener Materialien verwendet werden, um die Kontaktfläche 40 auszubilden. Über den elektrisch leitenden Faden 30 werden myoelektrische Signale von einem Patienten aufgenommen und über Leiter 50, die innerhalb des Grundkörpers 10 entlanggeführt sind, weitergeleitet. Die elektrisch leitenden Fäden 30 sind so angeordnet und an dem Grundkörper 10 festgelegt, dass sie jeweils eine separate Kontaktfläche 40 ausbilden, über die die myoelektrischen Signale von den jeweiligen Elektroden 21, 22, 23 aufgenommen und weitergeleitet werden kann. Der Zwischenraum zwischen den Elektroden 21, 22, 23 ist aus einem elektrisch nicht leitenden Material ausgebildet, so dass sich keine Signalverfälschungen ergeben. Durch die Gewebebildung mit dem Edelstahlgarn als elektrisch leitenden Faden 30 ist es möglich, dass man nicht durch alle Lagen des Grundkörpers 10 hindurchsticht, sondern dass die elektrisch leitenden Fäden 30 ausschließlich auf der Oberfläche der Innenseite 11 mit der Haut in Kontakt treten können. Dadurch muss auf der Außenseite des Grundkörpers 10 keine Isolierung aufgebracht werden. Die Leiter 50 können ebenfalls aus einem elektrisch leitenden Faden 30 ausgebildet sein.

Figur 2 zeigt die Außenseite 12 des Grundkörpers 10 mit einem darauf befestigten Verstärker oder Steckerelement 70. Die Leiter 50 innerhalb des Grundkörpers 10 führen von den nicht dargestellten Elektroden 21, 22, 23 auf der Innenseite 11 zu dem Verstärker oder Steckerelement 70 auf der Außenseite 12. , Die Leiter 50 können durch einen einfachen Vorstich im Zwischenraum des Grundkörpers 10, der beispielsweise als Abstandsgewebe ausgebildet sein kann, innerhalb des Grundkörpers 10 angeordnet werden. Die Leiter 50 werden an die Oberfläche der Außenseite 12 gebracht und mit entsprechenden Aufnahmen an dem Steckerelement 70 verbunden. Dies kann beispielsweise durch eine formschlüssige Verbindung des elektrischen Leiters 50 in einer Durchgangsbohrung oder einer Öse, ähnlich einem Nadelöhr erfolgen. Der elektrische Leiter 50 wird durch die Öffnung hindurchgeführt und verknotet. Alternativ oder ergänzend kann beispielsweise ein Stecken, Verlöten oder Crimpen des elektrischen Leiters mit dem Kontaktpunkt an dem Steckerelement 70 das myoelektrische Signal, gegebenenfalls nach einer Verstärkung durch einen an dem Steckerelement 70 angeordneten Verstärker zu einer Auswertung für eine Steuerungseinrichtung weitergeleitet werden. Anstelle des Steckerelementes 70 kann auch eine Steckerbasis oder ein anderes elektrisches oder elektronisches Bauteil auf diese Art und Weise mit den myoelektrischen Elektroden 21, 22, 23 auf der Innenseite 11 des Grundkörpers 10 verbunden werden, wobei die Komponenten bevorzugt auf der Außenseite 12 des Grundkörpers 10 liegen.

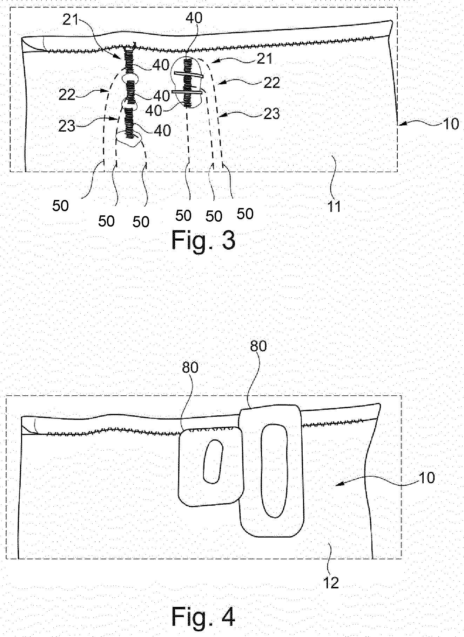

Figur 3 zeigt eine Variante der Erfindung mit einem Grundkörper 10 in Teildarstellung mit auf der Innenseite 11 angeordneten Elektroden 21, 22, 23. In dem dargestellten Ausführungsbeispiel sind drei Elektroden vorhanden, von denen die mittlere Elektrode 22 als Erdungselektrode ausgebildet ist. Auch hier sind zwischen den Elektroden 21, 22, 23 nicht leitende Freiräume vorhanden, wobei die Kontaktflächen 40 der Elektroden 21, 22, 23 wiederum durch einen elektrisch leitenden Faden 30 ausgebildet sind. In dem dargestellten Ausführungsbeispiel ist der elektrisch leitende Faden 30 ein Edelstahlgarn, das auf dem Grundkörper 10 aufgenäht ist. Das Aufnähen kann mit einem Unterfaden aus einem Edelstahlgarn und einem Oberfaden aus einem nicht leitenden Material, beispielsweise einem Polyestergarn erfolgen. Zwischen den Kontaktflächen 40 kann eine Folie angeordnet sein, um eine weitere Trennung zwischen den Elektroden 21, 22, 23 zu bewirken. Auch hier führen Leiter 50 von den einzelnen Elektroden 21, 22, 23 durch das Gewebe des Grundkörpers 10 zu einer weiteren Komponente, die Leitung 50 kann als einfacher Vorstich in dem Zwischenraum des Abstandsgewirkes des Grundkörpers 10 ausgebildet sein. Die Kontaktflächen 40 können mit einem Schnellnäher im Zickzack aufgenäht sein, der Anpressdruck kann durch auf der Rückseite aufgebrachte Volumenelemente, beispielsweise aus Silikon oder dergleichen, vergrößert werden. Diese Volumenelemente sind den Elektroden 21, 22, 23 auf der Außenseite hinterlegt und mit einer Bondingfolie 80 korrespondieren auf der Außenseite 12 angeordnet, wie in der Figur 4 dargestellt.

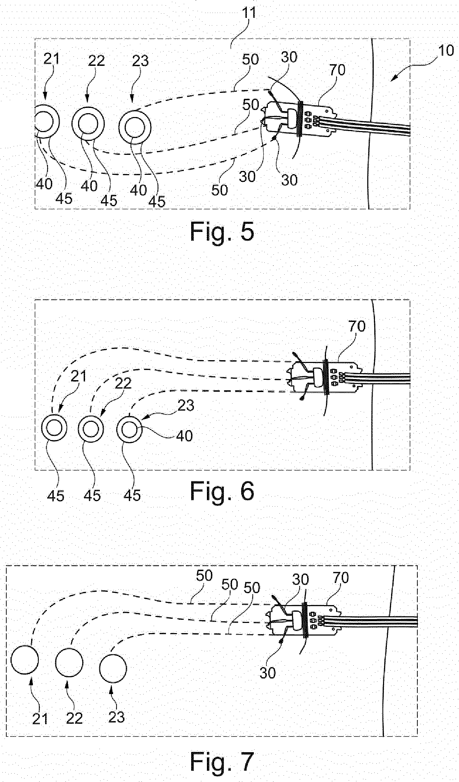

Figur 5 zeigt eine Variante der Ausgestaltung der Textilelektrode mit Kontaktflächen 40, die aus einem Zuschnitt 45 ausgebildet sind. Der Zuschnitt 45 weist zumindest im Bereich der Kontaktflächen 40 zumindest einen elektrisch leitenden Faden 30 auf, bevorzugt besteht der Zuschnitt 45 überwiegend aus elektrisch leitenden Fäden 30, zumindest auf der Oberseite oder der Seite, die die Kontaktfläche 40 mit der Haut bildet. Die Zuschnitte 45 können ausgeschnitten, ausgestanzt oder auf andere Art und Weise von einem Grundzuschnitt getrennt werden, in den dargestellten Ausführungsbespielen sind die Zuschnitte 45 im Wesentlichen rund. Der Zuschnitt 45 kann ebenfalls mit einem elektrisch leitenden Faden 30 bestickt sein. Um eine Erhöhung des Anpressdruckes der Elektroden 21, 22, 23 zu erreichen, wird ein Volumenelement dem Zuschnitt 45 hinterlegt und zusammen mit dem Zuschnitt 45 auf dem Grundkörper 10 festgelegt. Das Festlegen kann durch Verschweißen, Verkleben oder Vernähen stattfinden. Wenn das Vernähen mit einem elektrisch leitenden Faden erfolgt, kann dieser als Leiter 50 zu einem Steckerelement, Verstärker, einer Steckerbasis oder einer anderen elektrischen oder elektronischen Komponente benutzt werden.

Der elektrisch leitende Faden 30 wird dann aus dem Grundkörper 10 an eine Oberfläche herausgeführt, im dargestellten Ausführungsbeispiel an die Innenseite 11. Grundsätzlich ist auch möglich, den elektrisch leitenden Faden 30 auf der Außenseite 12 aus dem Grundkörper 10 herauszuführen.

In der Figur 6 ist eine Variante der Erfindung gezeigt, die im Wesentlichen der der Figur 5 entspricht, jedoch ist statt einer einlagigen Ausgestaltung des Zuschnittes 45 in der Figur 6 ein zweilagiger Zuschnitt vorgesehen, der über Verklebung oder eine Bondingfolie 80 zu einem eingeschlossenen Volumenelement dicker als die einlagige Variante ausgebildet ist. Durch die weiterhin flächige Auflage der unteren Lage ergibt sich eine stabile Ortszuordnung und eine leichte Vernähbarkeit.

Figur 7 zeigt eine weitere Variante mit kugelförmigen Elektroden 21, 22, 23, die durch ein kugelförmiges Volumenelement zusammen mit dem Zuschnitt 45 gebildet und auf den Grundkörper 10 aufgenäht wurden. In den Ausführungsformen gemäß Figuren 5 und 7 sind die Elektroden 21, 22, 23 mit Edelstahlgarn mit Vorstich umstickt, der elektrisch leitende Faden 30 führt als Leiter 50 von der Elektrode 21, 22, 23 direkt zu dem Steckelement 70 oder einem Verstärker.

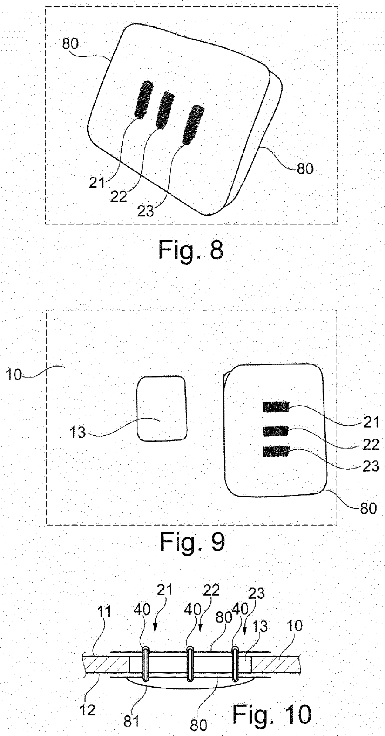

Figur 8 zeigt eine Variante der Erfindung, bei der die jeweilige Elektrode 21, 22, 23 auf zwei Lagen Bondingfolie 80 mit einem elektrisch leitenden Faden 30 aufgenäht wurde, um ein modular ausgebildetes Kontaktflächenpad zu bilden. Die Klebeschichten der Bondingfolien 80 sind einander zugewandt, die mittlere Elektrode 22 ist die Erdungselektrode. Der grundsätzliche Aufbau ist der Figur 10 zu entnehmen, die in der schematischen Schnittdarstellung die oben und unten angeordneten Bondingfolien 80, die Elektroden 21, 22, 23 nebeneinander mit den jeweiligen Kontaktflächen 40 und dem Grundkörper 10 mit einer Ausnehmung 13 zeigt. Diese Ausnehmung 13 ist in der Figur 9 zusammen mit dem Kontaktflächenpad zu erkennen. Zur Befestigung der Elektroden 21, 22, 23 an dem Grundkörper 10 wird das Kontaktflächenpad eingefädelt, so dass sich die Elektroden 21, 22, 23 innerhalb der Ausnehmung 13 befinden. An der Innenseite 11 und der Außenseite 12 ist der Überstand der jeweiligen Bondingfolie 80 aufgelegt und kann dort mit dem Grundkörper 10 verklebt, verschweißt, vernäht oder anderweitig fixiert werden. Auf der Außenseite 12 kann eine Schutzabdeckung 81 für elektrisch leitende Fäden 30 auf der Außenseite 12 aufgebracht sein.

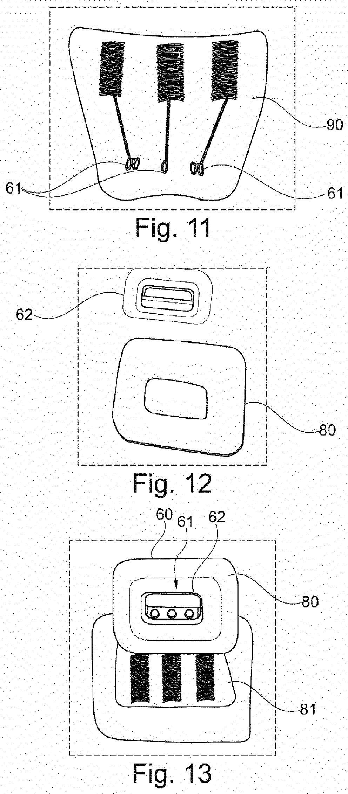

Figur 11 zeigt eine Variante der Figur 8, bei der auf ein Textilzuschnitt 90 über elektrisch leitende Fäden 30 auf der nicht dargestellten Unterseite Elektroden ausgebildet werden. Über nicht dargestellte Leiter werden diese Elektroden mit elektrisch leitenden Anschlusspunkten 61 verbunden. Um eine Steckerbasis 60 auszubilden, ist in der Figur 12 ein Aufnahmerahmen 62 gezeigt, der eine mechanische Festlegung eines nicht dargestellten Steckerelementes ermöglicht. Der Steckeraufnahmerahmen 62 wird zu den elektrisch leitenden Anschlusspunkten 61 zugeordnet und auf der Oberfläche des Textilzuschnittes 90 befestigt. Die Befestigung erfolgt über eine Bondingfolie 80, die einen entsprechend großen Ausschnitt zum Durchtritt des Steckerelementes zu den Anschlusspunkten 61 gewährleistet. Eine fertigmontierte Steckerbasis 60 ist in der Figur 13 gezeigt, der Steckerbasisrahmen 62 mit den darin angeordneten Anschlusspunkten 61 ist ebenso zu erkennen wie die Bondingfolie 80 und die isolierend durch die Abdeckung 81 geschützten elektrischen Fäden der Elektroden.

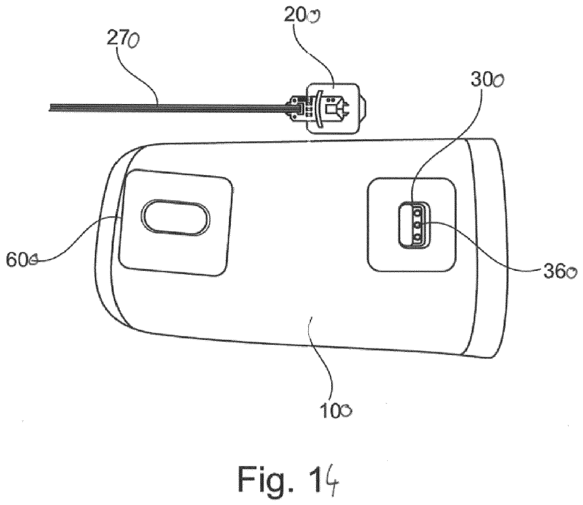

Figur 14 zeigt eine Draufsicht einer orthopädietechnischen Einrichtung in Gestalt eines Liners mit einem textilen Grundkörper 100, der beispielsweise aus einem Abstandsgewirk oder einem mehrlagigen Textil bestehen kann. Ebenfalls ist es möglich, dass der Grundkörper 100 aus einem Schaumstoff oder einem Elastomer besteht, beispielsweise Polyurethan oder Silikon. Ebenfalls ist es möglich, dass der Grundkörper 100 eine Kombination von Elastomer und Textil aufweist, beispielsweise eine Innenbeschichtung oder eine Außenbeschichtung mit einem textilen Werkstoff und auf der jeweils gegenüberliegenden Seite eine Ausgestaltung mit einem Elastomerwerkstoff.

An der Außenseite des Grundkörpers 100 ist eine Basis 300 mit einer Öffnung 350 zur Aufnahme eines Steckers 200 angeordnet. Innerhalb der Basis 300 sind Kontaktflächen 360 angeordnet, die mit nicht dargestellten Steckerkontakten in Verbindung bringbar sind, wenn der Stecker 200 in die Basis 300 eingeführt wird. Von dem Stecker 200 führen Kabel oder Leiter 270 zu einer elektrischen oder elektronischen Komponente, beispielsweise eine Steuereinrichtung, Auswerteeinrichtung oder dergleichen. Beabstandet von der Basis 300 ist eine Elektrode 600 innerhalb des Grundkörpers 300 eingearbeitet. Die Elektrode 600 reicht bis auf die nicht dargestellte Innenseite des Grundkörpers 100 und nimmt beispielsweise biometrische Signale oder insbesondere myoelektrische Signale auf. Innerhalb des Grundkörpers 100 sind nicht dargestellte Leiter angeordnet, insbesondere eingebettet, um myoelektrische Signale von den Elektroden 600 zu den Kontaktflächen 360 innerhalb der Basis 300 zu leiten.

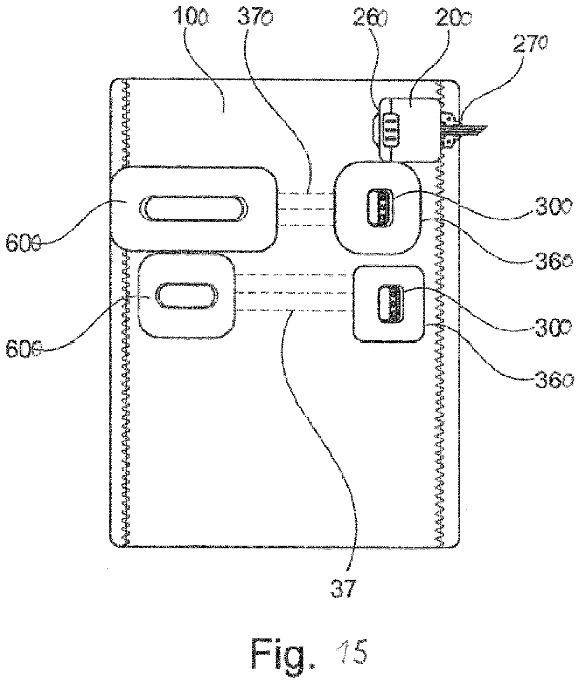

Figur 15 zeigt eine Variante der Figur 14 mit zwei Elektroden 600, wobei die elektrisch leitenden, mit der Hautoberfläche in Kontakt stehenden Elemente der Elektrode 600 nicht dargestellt sind. Die Außenseite der Elektroden 600 ist abgedeckt, beispielsweise mit einer Bondingfolie. Die Leiter 370 von den Elektroden 600 zu den beiden Basen 300 sind durch eine Strichlinie angedeutet. Innerhalb beider Basen 300 sind Kontaktflächen 360 angeordnet oder ausgebildet, die zu Kontaktflächen 260 an der Stirnseite des Steckers 270 korrespondieren. Im gefügten Zustand von Stecker 200 mit der Basis 300 liegen die Kontaktflächen 360 an den Steckerkontaktflächen 260 an. Von dem Stecker 200 werden die aufgenommenen Signale über Leitungen 270 weitergeleitet.

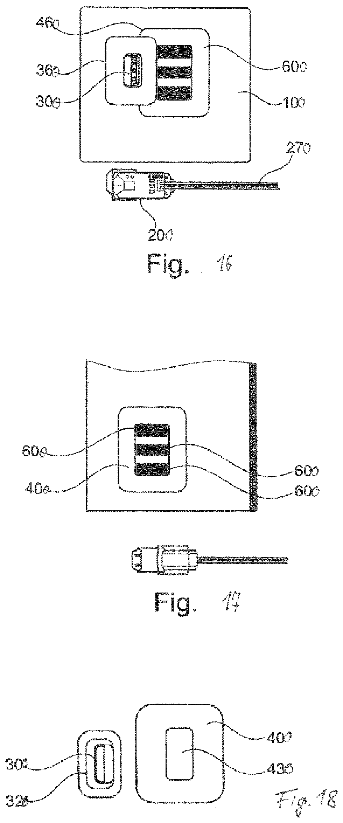

Figur 16 zeigt eine vergrößerte Darstellung der Basis 300 an der Außenseite eines Grundkörpers 100. Oberhalb des Steckers 200, der nicht in der Basis 300 eingefügt ist, ist eine weitere elektronische Komponente angeordnet, beispielsweise ein Verstärker 500. Die Basis 300 ist über eine Bondingfolie als Verbindungselement 400 auf dem Grundkörper 100 festgelegt, beispielsweise durch Verkleben. In unmittelbare Nähe zu der Basis 300 mit der Öffnung und den Kontaktflächen 360 sind die Sensoren oder Elektroden 600 in Gestalt von aufgestickten, aufgenähten, aufgedruckten oder anderweitig aufgebrachten elektrisch leitenden Fäden angeordnet. Die Elektroden 600 können durch Durchnähen elektrisch leitender Fäden durch den Grundkörper 100 auf einfache Art und Weise hergestellt werden. Von den Elektroden 600, die myoelektrische Sensoren darstellen, wird unmittelbar ein Leiter zu den Kontaktpunkten oder Kontaktflächen 360 geführt. Nach außen hin sind die elektrisch leitenden Fäden der Elektroden 600 durch die Bondingfolie 400 abgedeckt.

Figur 17 zeigt eine Elektrodenanordnung in Untenansicht mit freien, auf der Haut auflegbaren Elektroden 600 auf einer Bondingfolie 400.

Figur 18 zeigt die Basis 300 mit einem Auflagerand 320 im noch nicht gefügten Zustand. Der Auflagerand 320 kann mit dem Grundkörper 100 verbunden werden, beispielsweise vernäht, verklebt oder verschweißt, gegebenenfalls nur bereichsweise verklebt, vernäht oder verschweißt. Über die Basis 300 mit der daran ausgebildeten Öffnung zur Aufnahme des Steckers wird das Verbindungselement 400 in Gestalt einer Bondingfolie gelegt und anschließend mit dem nicht dargestellten Grundkörper verbunden. Innerhalb des Verbindungselementes 400 ist ein Ausschnitt 430 ausgebildet, um einen freien Zugang zu der Basis 300 zu erlauben.

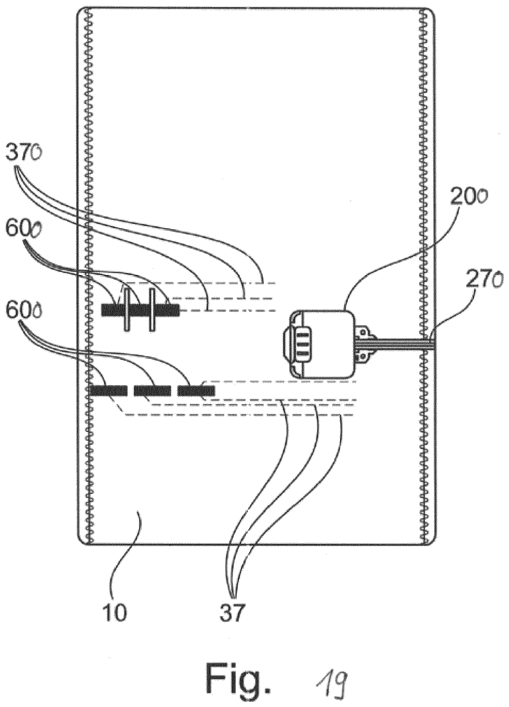

Figur 19 zeigt die Ausgestaltung von zwei Elektrodenanordnungen, wie sie beispielsweise in der Figur 15 von der Außenseite zu erkennen sind, von einer Unterseite. Jeweils zwei Elektroden 600 mit drei durch elektrisch leitende Fäden gebildete Aufnahmebereiche, die elektrisch voneinander isoliert sind, sind auf der Unterseite des Grundkörpers 100 ausgebildet. Über elektrische Leiter, die innerhalb des Grundkörpers 100 entlanggeführt sind, werden die Elektroden 600 mit den auf der Außenseite angeordneten Basen verbunden, um dann mit dem Stecker 200 verbunden zu werden.

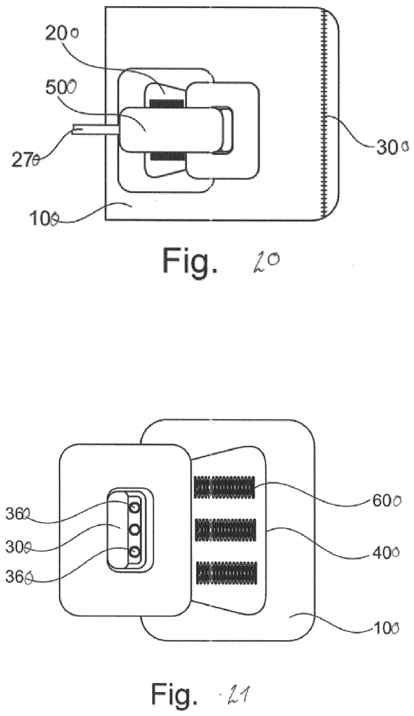

Figur 20 zeigt eine orthopädietechnische Einrichtung mit einer fertig montierten Kontaktierungseinrichtung mit der Basis 300 und dem darin angeordneten Stecker 200, auf dessen Oberseite ein Verstärker 500 angeordnet ist.

Figur 21 zeigt in einer Detailansicht die durch die Bondingfolien 400 fixierten Elektroden 600, die in Verbindung mit der Basis 300 und den innerhalb der Basis ausgebildeten Kontaktflächen 360 stehen.

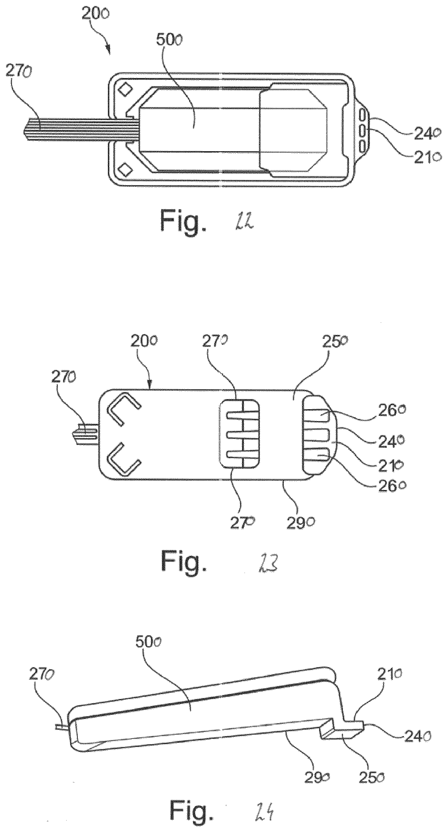

Figur 22 zeigt in einer Einzeldarstellung ein Steckerelement 200 mit einem Verstärker 500 oder einer anderen elektronischen Einrichtung auf der Oberseite sowie einem Formschlusselement 210 in Gestalt einer vorspringenden Zunge, an dessen Vorderkante 240 Steckerkontaktflächen angeordnet sein können. Das Steckerelement 200 weist eine im Wesentlichen rechteckige Formgebung auf, mit einem Absatz von einer Grundfläche, sodass der Stecker über diesen Absatz mit der Basis in formschlüssigen Kontakt treten kann.

Figur 23 zeigt den Stecker gemäß Figur 22 in einer Untenansicht, die Leiter 270 sind ebenso zu erkennen wie die an der Unterseite 250 der Zunge 210 angeordneten Steckerkontaktflächen 260, die etwas zurück von der Vorderkante 240 stehen. Ein Absatz 290 ist zu erkennen, der sich quer zu der Längserstreckung des Steckerelementes 200 erstreckt und im Wesentlichen parallel zu der Vorderkante 240 der Zunge 210 verläuft.

In der Seitenansicht gemäß Figur 24 ist der Absatz 290 auf der der Stirnseite 240 gegenüberliegenden Seite des Formschlusselementes 210 zu erkennen. Über die Unterseite 250 stehen die Steckerkontaktflächen 260 hinaus.

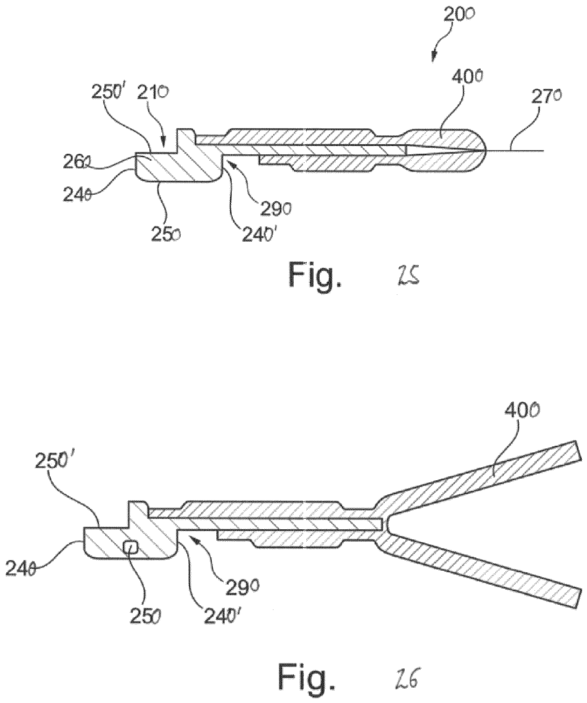

Figur 25 zeigt eine schematische Schnittdarstellung einer Ausführungsform eines Steckerelementes 200 mit einem vorspringenden Formschlusselement 210 in Gestalt einer Zunge, dessen Stirnseite 240 eine rückwärtige Stirnseite 240' gegenüberliegt. Die Zunge 210 bildet einen zur Unterseite 250 vorspringenden Absatz 290 aus, ebenso ist auf der Oberseite 250' ein Absatz ausgebildet, um einen Anschlag gegen ein zu weites Hineinschieben in die Basis auszubilden. An der Stirnseite 240 können Steckerkontaktflächen 260 angeordnet oder ausgebildet sein, alternativ oder ergänzend können solche Steckerkontaktflächen 260 an der Unterseite 250 des Steckerelementes 200 angeordnet sein. Eine Leitung 270 ist angedeutet, die durch das Steckerelement 200 von den Steckerkontaktflächen 260 in eine rückwärtige Richtung führen. Die Leiter 270 sind über ein Verbindungselement 400 an dem Steckerelement 200 festgelegt und eingekapselt.

Figur 26 zeigt ein Steckerelement 200 vor dem Verbinden beispielsweise mit einem Band oder anderen Leitern 270, die nicht dargestellt sind. Dazu werden die Bondingfolien 400 als Verbindungselemente aufeinandergelegt und gegebenenfalls verschweißt.

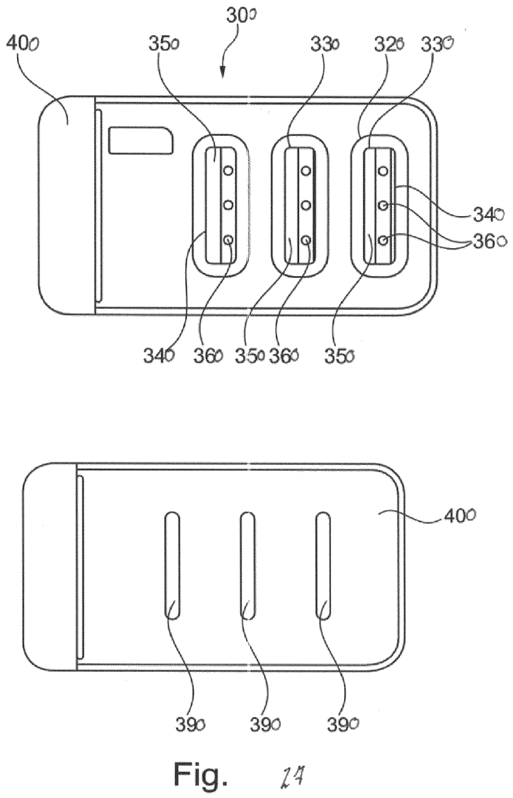

Figur 27 zeigt ein Ausführungsbeispiel einer Basis in Draufsicht in der oberen Darstellung und in einer Untenansicht in der unteren Darstellung. Insgesamt sind drei Öffnungen 35 innerhalb der Basis 300 ausgebildet, die im Wesentlichen parallel zueinander und rechtwinklig orientierte Seitenkanten 340, 330 aufweisen. Zum leichteren Fügen sind die Seitenkanten 330, 340 in ihren Übergangsbereichen abgerundet. Innerhalb der Basis 300 sind auf dem Boden der Basis 300 und durch die Öffnung 350 von oben zugänglich Kontaktflächen 360 angeordnet, die mit nicht dargestellten Leitern verbunden sind. Die jeweilige Basis 300 weist einen Auflagerand 320 auf, der verschweißbar, verklebbar oder auf andere Art und Weise verbindbar mit dem Verbindungselement 400 verbunden ist, das eine größere Auflagefläche bereitstellt, um auf dem Grundkörper 100 festgelegt zu werden. Grundsätzlich ist es auch möglich, ohne Verbindungselemente 400 die Basis 300 mit dem Auflagerand 320 an der Oberfläche der jeweiligen Grundkörper 100 anzuordnen.

In der unten Darstellung ist die geschlossene Oberfläche der jeweiligen Basis zu erkennen, sodass von unten, also von der Innenseite der orthopädietechnischen Einrichtung, kein Schmutz in die Öffnung 350 eindringen kann. Schweißnähte 390 oder Klebenähte können an der Unterseite aufgebracht sein, um eine Verbindung mit dem nicht dargestellten Grundkörper zu erleichtern.

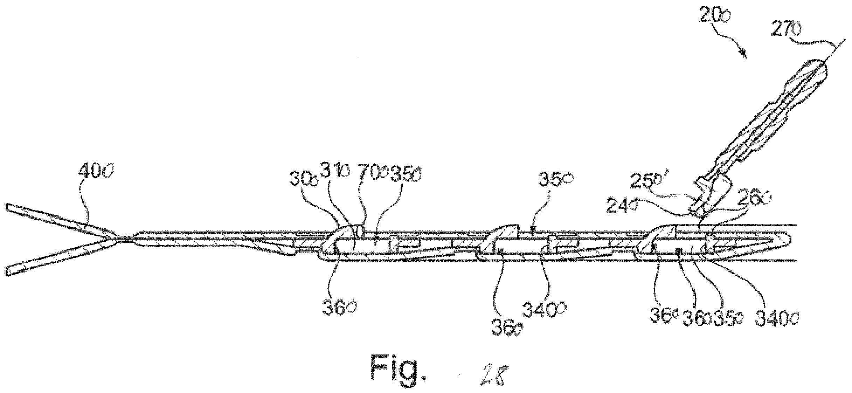

Figur 28 zeigt eine schematische Darstellung eines Fügevorganges der Steckerelemente 600 mit einer Basis 300. In dem dargestellten Ausführungsbeispiel sind drei Basen 300 angeordnet, grundsätzlich reicht eine Basis aus. In der linken Basis 300 sind die Kontaktflächen 360 an einer Stirnseite einer Ausnehmung oder Öffnung des Hinterschnittes 310 angeordnet, in der mittleren sind sie an der Unterseite der Öffnung 350 angeordnet, in der rechten Darstellung sowohl an der Stirnseite als auch an der Unterseite, sodass eine Kontaktierung sowohl durch Druck von oben als auch durch Druck in Einführrichtung gefördert wird. In der linken Darstellung der Basis 300 ist eine Dichtung 700 angedeutet, die zur Abdichtung im gefügten Zustand dient. Die Dichtung 700 kann umlaufend um die Öffnung 350 angeordnet sein.

Um das Steckerelement 200 in die Basis 300 einzuführen, ist am Übergang von der Stirnseite 240 zur Unterseite 250 eine Abschrägung ausgebildet, sodass das Steckerelement 200 unter Ausbildung einer leichten Schräge in die Öffnung 350 eingeführt und wie durch den Pfeil angedeutet unter den Hinterschnitt 310 eingeschoben und eingeschwenkt wird, bis die Stirnseite 240 gegen die vorderen Kontaktflächen 360 drücken. Die Dicke des Steckerelementes 200 im Bereich der Zunge 210 ist so bemessen, dass sie in die Basis 300 einführbar ist. Die Steckerkontaktflächen 260 können federbelastet und elastisch ausgebildet sein, um eine ausreichende Anpresskraft bereitzustellen. Die rückwärtige Seitenwand 3400 der Öffnung 300 kann federnd vorgespannt oder durch elastische Verformung beim Einführen so verformt werden, dass sie im gefügten Zustand einen nach vorne gerichteten Druck ausübt, sodass das Steckerelement 200 rastend gehalten wird. Ebenfalls ist es möglich, dass die Rückwand 3400 eine Neigung aufweist, die korrespondierend zu einer Neigung der rückwärtigen Stirnwand 240' ausgebildet ist. Die Neigung der Stirnwand 3400 ist nach vorne gerichtet, sodass neben einer elastischen Vorspannung auch eine formschlüssige Verriegelung des Steckerelementes 200 in der Basis 300 erfolgen kann.

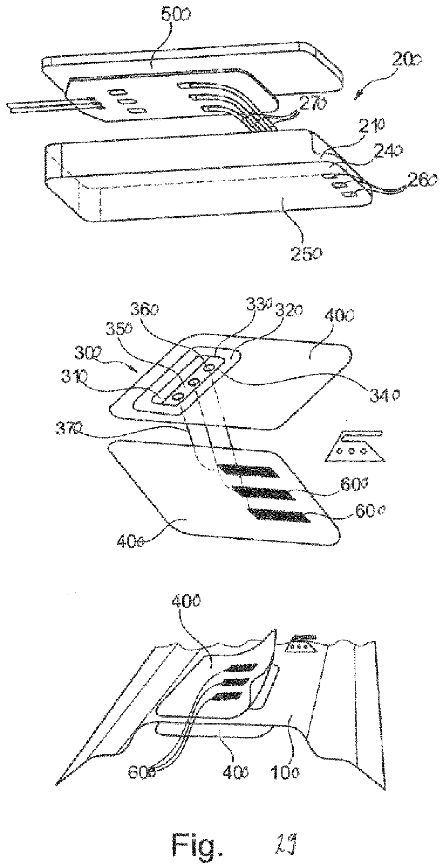

Figur 29 zeigt den schematischen Aufbau der Kontaktierungseinrichtung mit dem Steckerelement 200, das ein Grundelement aufweist, das an seiner Unterseite Steckerkontaktflächen 260 aufweist. Das Grundelement weist eine nach vorne hervorstehende Zunge 210 als Formschlusselement auf. Von den Steckerkontaktflächen 260 führen elektrische Leiter 270 zu einem Verstärker 500, der auf der Oberseite des Steckerelementes 200 angeordnet ist.

Die Basis 300 weist einen Hinterschnitt 310 auf, auf dem Grund der Basis 300 sind drei Kontaktflächen 360 angeordnet, die korrespondierend zu den Steckerkontaktflächen 260 des Steckerelementes ausgebildet und positioniert sind, sodass im gefügten Zustand eine einseitige elektrische Kontaktierung vorhanden ist. Insgesamt sind drei Steckerkontaktflächen 260 und drei Kontaktflächen 360 vorgesehen, abweichende Anzahlen können nach Notwendigkeit vorgesehen sein. Die Öffnung 350, durch die die Kontaktflächen 360 zugänglich sind, ist durch die im Wesentlichen rechtwinklig zueinander orientierten Seitenflächen 330, 340 ausgebildet, um die sich herum ein Auflagerand 320 erstreckt, um die Basis 300 mit dem Verbindungselement 400 in Gestalt einer Bondingfolie zu verbinden. Die Leiter 370 werden auf eine zweite Bondingfolie 400 geführt und dort entweder mit Sensoren oder Elektroden 600 verbunden oder aufgenäht, um dort die Elektroden 600 auszubilden.

In der unteren Darstellung der Figur 29 ist der Fügeprozess gezeigt, mit der die Basis 300 an dem Grundkörper 100 festgelegt werden kann. Die beiden Bondingfolien 400, die in der mittleren Darstellung gezeigt sind, werden auf der Oberseite und Unterseite des Grundkörpers 100 positioniert. Die Leiter 370 treten durch einen Ausschnitt innerhalb des Grundkörpers 100 hindurch. Die Basis 300 mit der Öffnung ist im dargestellten Ausführungsbeispiel auf der Unterseite angeordnet und nicht zu erkennen. Anschließend wird durch Aufbringen von Druck und thermischer Energie, wie durch ein Bügeleisen angedeutet, eine Verklebung bereitgestellt, sodass die Basis 300 zusammen mit den Elektroden 600 aufgebracht wird.

Alternativ kann durch elektrisch leitende Fäden ein Kontaktfeld aufgebaut werden, das über die Bondingfolien mit dem Grundkörper 100 verbunden wird. Es kann beispielsweise leitendes Garn als Elektroden über ein MPU-Bondingfilm auf den Grundkörper 100 aufgebracht werden. Korrespondierende Kontaktflächen werden an der Bondingfolie, die mit der Basis 300 verbunden ist, ausgebildet und zum einfachen Fügen werden die jeweiligen Bondingfolien miteinander verbunden.

## Patentansprüche

1. Textilelektrode mit einem flächigen Grundkörper (10) aus einem elektrisch nicht leitenden Material mit einer im angelegten Zustand einer Hautoberfläche zugewandten Innenseite (11) und einer der Innenseite (11) gegenüberliegenden Außenseite (12) wobei zumindest eine myoelektrische Signale aufnehmende Elektrode (21, 22, 23) auf der Innenseite (11) angeordnet oder ausgebildet ist, wobei die Elektrode (21, 22, 23) zumindest einen elektrisch leitenden Faden (30) aufweist oder daraus ausgebildet ist, der eine Kontaktfläche (40) ausbildet, wobei die Kontaktfläche (40) auf einer Bondingfolie (80) ausgebildet ist, die über den Außenumfang der Kontaktfläche (40) hinaussteht und die Elektrode (21, 22, 23) oder ein Textilzuschnitt (90) in einen dazu korrespondierenden Ausschnitt (13) in dem Grundkörper (10) eingelegt und über die Bondingfolie (80) daran befestigt ist und die Außenseite der Elektrode (21, 22, 23) durch eine weitere Bondingfolie (80) abgedeckt ist.

2. Textilelektrode nach Anspruch 1, **dadurch gekennzeichnet, dass** die Elektrode (21, 22, 23) auf dem Grundkörper (10) aufgestickt, aufgenäht, eingestrickt, eingewebt, aufgeklebt, gedruckt, gebonded, aufgeschweißt, eingeschweißt oder als integraler Bestandteil des Grundkörpers (10) ausgebildet ist.

3. Textilelektrode nach Anspruch 1 oder 2, **dadurch gekennzeichnet, dass** die Elektrode (21, 22, 23) mit einem metallischen Ober- und/oder Unterfaden als elektrisch leitenden Faden (30) ausgebildet ist.

4. Textilelektrode nach einem der voranstehenden Ansprüche, **dadurch gekennzeichnet, dass** die Elektrode (21, 22, 23) mit einem Lege- und/oder Nadelfaden als elektrisch leitenden Faden (30) ausgebildet ist.

5. Textilelektrode nach einem der voranstehenden Ansprüche, **dadurch gekennzeichnet, dass** die Elektrode (21, 22, 23) als als Fläche verstrickter elektrisch leitender Faden (30) ausgebildet ist.

6. Textilelektrode nach einem der voranstehenden Ansprüche, **dadurch gekennzeichnet, dass** die Elektrode (21, 22, 23) mit einem Kett- und/oder Schussfaden als elektrisch leitenden Faden (30) ausgebildet ist.

7. Textilelektrode nach einem der voranstehenden Ansprüche, **dadurch gekennzeichnet, dass** mehrere Kontaktflächen (40) nebeneinander und elektrisch voneinander getrennt an dem Grundkörper (10) angeordnet sind.

8. Textilelektrode nach einem der voranstehenden Ansprüche, **dadurch gekennzeichnet, dass** von der Kontaktfläche (40) ein elektrischer Leiter (50) zu einer elektrischen oder elektronischen Komponente, einer Steckerbasis (60) oder einem Stecker (70) an oder in dem Grundkörper (10) geführt ist.

9. Textilelektrode nach einem der voranstehenden Ansprüche, **dadurch gekennzeichnet, dass** die Kontaktfläche (40) aus einem Zuschnitt (45) ausgebildet ist, die an dem Grundkörper (10) befestigt ist.

10. Textilelektrode nach Anspruch 9, **dadurch gekennzeichnet, dass** der Zuschnitt (45) mit einem Volumenelement hinterlegt ist.

11. Textilelektrode nach einem der voranstehenden Ansprüche, **dadurch gekennzeichnet, dass** der Grundkörper (10) aus einem Abstandstextil mit einem Obertextil, einem Untertextil und dazwischen angeordneten Stützfäden, die das Obertextil und das Untertextil aneinander festlegen und zueinander beabstandet halten.

12. Textilelektrode nach Anspruch 1, **dadurch gekennzeichnet, dass** die Kontaktfläche (40) auf einem Textilzuschnitt (90) mit einer zumindest auf einer Oberfläche angeordneten Bondingfolie (80) ausgebildet ist, die über den Außenumfang des Textilzuschnittes (90) übersteht und mit dem Grundkörper (10) verbunden ist.

13. Textilelektrode nach einem der voranstehenden Ansprüche, **dadurch gekennzeichnet, dass** von der Kontaktfläche (40) ein elektrischer Leiter (50) zu einem auf der Außenseite (12) des Grundkörpers (10) oder eines Textilzuschnittes (90) angeordneten, elektrisch leitenden Anschlusspunkt (61) führt.

## Claims

1. Textile electrode with a flat base body (10) made of an electrically non-conductive material with an inner side (11) facing a skin surface when applied and an outer side (12) opposite the inner side (11), wherein at least one electrode (21, 22, 23) for receiving myoelectric signals is arranged or formed on the inner side (11), wherein the electrode (21, 22, 23) has at least one electrically conductive thread (30) or is formed therefrom, which forms a contact surface (40), wherein the contact surface (40) is formed on a bonding film (80) which protrudes beyond the outer circumference of the contact surface (40) and the electrode (21, 22, 23) or a textile cut-out (90) is inserted into a corresponding cut-out (13) in the base body (10) and is attached thereto via the bonding foil (80) and that the outside of the electrode (21, 22, 23) is covered by a further bonding foil (80).

2. Textile electrode according to claim 1, **characterised in that** the electrode (21, 22, 23) is embroidered, sewn, knitted, woven, glued, printed, bonded, welded, heat-sealed or formed as an integral part of the base body (10).

3. Textile electrode according to claim 1 or 2, **characterised in that** the electrode (21, 22, 23) is formed with a metallic upper thread and/or lower thread as electrically conductive thread (30).

4. Textile electrode according to one of the preceding claims, **characterised in that** the electrode (21, 22, 23) is formed with a weft and/or warp thread as an electrically conductive thread (30).

5. Textile electrode according to one of the preceding claims, **characterised in that** the electrode (21, 22, 23) is designed as a surface of interwoven electrically conductive thread (30).

6. Textile electrode according to one of the preceding claims, **characterised in that** the electrode (21, 22, 23) is formed with a warp and/or weft thread as an electrically conductive thread (30).

7. Textile electrode according to one of the preceding claims, **characterised in that** several contact surfaces (40) are arranged next to each other and electrically separated from each other on the base body (10).

8. Textile electrode according to one of the preceding claims, **characterised in that** an electrical conductor (50) is led from the contact surface (40) to an electrical or electronic component, a plug base (60) or a plug (70) on or in the base body (10).

9. Textile electrode according to one of the preceding claims, **characterised in that** the contact surface (40) is formed from a cut piece (45) which is attached to the base body (10).

10. Textile electrode according to claim 9, **characterised in that** the cut piece (45) is backed with a volume element.

11. Textile electrode according to one of the preceding claims, **characterised in that** the base body (10) is made of a spacer textile with an upper textile, a lower textile and support threads arranged between them, which fix the upper textile and the lower textile to each other and keep them spaced apart.

12. Textile electrode according to claim 1, **characterised in that** the contact surface (40) is formed on a textile cut piece (90) with a bonding film (80) arranged at least on one surface, which protrudes beyond the outer circumference of the textile cut piece (90) and is connected to the base body (10).

13. Textile electrode according to one of the preceding claims, **characterised in that** an electrical conductor (50) leads from the contact surface (40) to an electrically conductive connection point (61) arranged on the outside (12) of the base body (10) or a textile cut piece (90).

## Revendications

1. Électrode textile comprenant un corps de base surfacique (10) en un matériau électriquement non conducteur, ayant une face intérieure (11) qui, à l'état appliqué, est tournée vers la surface de la peau, et une face extérieure (12) qui est opposée à la face intérieure (11),
dans laquelle
au moins une électrode (21, 22, 23) recevant des signaux myoélectriques (21, 22, 23) est disposée ou réalisée sur la face intérieure (11),
l'électrode (21, 22, 23) comprend au moins un fil électriquement conducteur (30) ou est réalisée à partir de celui-ci, qui constitue une surface de contact (40),
la surface de contact (40) est réalisée sur une feuille de métallisation (80) qui dépasse du pourtour extérieur de la surface de contact (40), et l'électrode (21, 22, 23) ou un flan textile (90) est inséré(e) dans une découpe correspondante (13) dans le corps de base (10) et est fixé(e) à celui-ci par l'intermédiaire de la feuille de métallisation (80), et la face extérieure de l'électrode (21, 22, 23) est recouverte par une autre feuille de métallisation (80).

2. Électrode textile selon la revendication 1,
**caractérisée en ce que** l'électrode (21, 22, 23) est brodée, cousue, tricotée, tissée, collée, imprimée, liée par métallisation, soudée sur ou soudée dans le corps de base (10) ou est réalisée comme partie intégrante du corps de base (10).

3. Électrode textile selon la revendication 1 ou 2,
**caractérisée en ce que** l'électrode (21, 22, 23) est réalisée avec un fil métallique supérieur et/ou inférieur comme fil électriquement conducteur (30).

4. Électrode textile selon l'une des revendications précédentes,
**caractérisée en ce que** l'électrode (21, 22, 23) est réalisée avec un fil de jetage et/ou un fil d'aiguille comme fil électriquement conducteur (30).

5. Électrode textile selon l'une des revendications précédentes,
**caractérisée en ce que** l'électrode (21, 22, 23) est réalisée comme un fil électriquement conducteur (30) tricoté en forme de surface.

6. Électrode textile selon l'une des revendications précédentes,
**caractérisée en ce que** l'électrode (21, 22, 23) est réalisée avec un fil de chaîne et/ou de trame comme fil électriquement conducteur (30).

7. Électrode textile selon l'une des revendications précédentes,
**caractérisée en ce que** plusieurs surfaces de contact (40) sont disposées sur le corps de base (10) en étant placées les unes à côté des autres et séparées électriquement les unes des autres.

8. Électrode textile selon l'une des revendications précédentes,
**caractérisée en ce qu'**un conducteur électrique (50) est mené depuis la surface de contact (40) vers un composant électrique ou électronique, une base de connecteur (60) ou un connecteur (70) sur ou dans le corps de base (10).

9. Électrode textile selon l'une des revendications précédentes,
**caractérisée en ce que** la surface de contact (40) est réalisée à partir d'un flan (45) qui est fixé au corps de base (10).

10. Électrode textile selon la revendication 9,
**caractérisée en ce que** le flan (45) est doublé d'un élément volumique.

11. Électrode textile selon l'une des revendications précédentes,
**caractérisée en ce que** le corps de base (10) est constitué d'un textile d'écartement comprenant un textile supérieur, un textile inférieur et des fils de soutien disposés entre ceux-ci, qui fixent le textile supérieur et le textile inférieur l'un à l'autre et les maintiennent à distance l'un de l'autre.

12. Électrode textile selon la revendication 1,
**caractérisée en ce que** la surface de contact (40) est réalisée sur un flan textile (90) comprenant une feuille de métallisation (80), disposée au moins sur une surface, qui dépasse du pourtour extérieur du flan textile (90) et est reliée au corps de base (10).

13. Électrode textile selon l'une des revendications précédentes,
**caractérisée en ce qu'**un conducteur électrique (50) est mené depuis la surface de contact (40) vers un point de connexion électriquement conducteur (61) disposé sur la face extérieure (12) du corps de base (10) ou d'un flan textile (90).
